# EUROPEAN PATENT SPECIFICATION

(11) **EP 3 151 897 B1**
(45) Date of publication and mention of the grant of the patent: **07.10.2020**
(21) Application number: 15729053.7
(22) Date of filing: 05.06.2015
(51) Int. Cl.: A61M 25/00, A61M 25/01, A61M 25/06

(54) **DELIVER ASSIST DEVICE FOR GUIDE CATHETER**
FREISETZUNGSUNTERSTÜTZUNGSVORRICHTUNG FÜR EINEN FÜHRUNGSKATHETER
DISPOSITIF D'ASSISTANCE DE POSE POUR CATHÉTER DE GUIDAGE

(30) Priority: 09.06.2014 US 201462009727 P
(43) Date of publication of application: 12.04.2017
(73) Proprietor: Boston Scientific Scimed Inc., Maple Grove, Minnesota 55311 (US)
(72) Inventor: WASDYKE, Joel M., Eden Prairie, Minnesota 55347 (US); MICKLEY, Timothy J., Corcoran, Minnesota 55340 (US)
(74) Representative: Vossius & Partner Patentanwälte Rechtsanwälte mbB
(86) International application number: PCT/US2015/034459
(87) International publication number: WO 2015/191393

(56) References cited:
- US-A- 5 395 332
- US-A1- 2002 119 264
- US-A1- 2011 295 217
- US-A1- 2012 209 284

## Description

### Cross-Reference To Related Applications

This application claims priority under 35 U.S.C. §119 to U.S. Provisional Application Serial No. 62/009,727, filed June 9, 2014.

### Technical Field

The present disclosure pertains to medical devices, and methods for manufacturing medical devices. More particularly, the present disclosure pertains to elongated intracorporeal medical devices including a tubular member connected with other structures, and methods for manufacturing and using such devices.

### Background

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

### Brief Summary

The invention is defined in the claims. This disclosure provides design, material, manufacturing method, and use alternatives for medical devices such as deliver assist devices and guide catheter assemblies. A deliver assist device for use in combination with a guide catheter is disclosed. The deliver assist device comprises:
an elongate proximal shaft;
a flexible distal section extending distally from the elongate proximal shaft, the flexible distal section including at least a first portion having a first flexibility and a second portion having a second flexibility;
a lumen extending through the flexible distal section, the lumen configured to accommodate a guidewire extendible therethrough; and
a tapered distal tip extending distally from the flexible distal section.

The first portion is formed of a polymeric material having a first durometer and the second portion is formed of the same polymeric material but having a second durometer different from the first durometer.

Alternatively or additionally to any of the embodiments above, the flexible distal section further comprises a third portion having a third flexibility.

Alternatively or additionally to any of the embodiments above, the third portion is formed of the same polymeric material but having a third durometer different from the first durometer and the second durometer.

Alternatively or additionally to any of the embodiments above, the third portion is formed of a second polymeric material different from the polymeric material of the first and second portions.

Alternatively or additionally to any of the embodiments above, the flexible distal section is configured to provide a step-wise change in flexibility between the first portion and the second portion.

Alternatively or additionally to any of the embodiments above, the flexible distal section comprises a tubular structure extending from the elongate proximal shaft.

Alternatively or additionally to any of the embodiments above, the flexible distal section comprises a widened portion of a tubular structure forming both the elongate proximal shaft and the flexible distal section.

Alternatively or additionally to any of the embodiments above, the distal tip has a distal tip flexibility different from the first flexibility or the second flexibility.

An assembly is disclosed. The assembly comprises:
a guide catheter extending between a distal region and a proximal region;
a guide catheter lumen extending from the distal region to the proximal region; and
a deliver assist device disposable within the guide catheter lumen, the deliver assist device including:
   an elongate proximal shaft;
   a flexible distal section extending distally from the elongate proximal shaft, the flexible distal section including at least a first portion having a first flexibility and a second portion having a second flexibility;
   a guidewire lumen extending through the flexible distal section; and
   a tapered distal tip extending distally from the flexible distal section.

Alternatively or additionally to any of the embodiments above, the deliver assist device is configured to be disposed within the guide catheter lumen for advancement of the assembly and then be removed from the guide catheter lumen once the assembly has reached a desired location within a vasculature.

Alternatively or additionally to any of the embodiments above, the deliver assist device, when disposed within the guide catheter lumen for advancement of the assembly, stiffens the guide catheter.

Alternatively or additionally to any of the embodiments above, the flexible distal section comprises a tubular structure extending from the elongate proximal shaft.

Alternatively or additionally to any of the embodiments above, the flexible distal section comprises a widened portion of a tubular structure forming both the elongate proximal shaft and the flexible distal section.

Alternatively or additionally to any of the embodiments above, the flexible distal section further comprises a third portion having a third flexibility.

A method of reaching a patient's cardiac vasculature is disclosed. The method comprises:
obtaining access to an artery in the patient's wrist;
advancing a guidewire through the artery to a desired location within the cardiac vasculature;
providing a deliver assist device in combination with a guide catheter;
advancing the deliver assist device in combination with the guide catheter over the guidewire to the desired location within the cardiac vasculature; and
withdrawing the deliver assist device so that a subsequent device may be delivered through the guide catheter.

Alternatively or additionally to any of the embodiments above, providing a deliver assist device in combination with a guide catheter comprises providing a deliver assist device having an elongate proximal shaft, a distal shaft section including a first portion having a first flexibility and a second portion having a second flexibility, and a tapered distal tip extending distally from the distal shaft section.

Alternatively or additionally to any of the embodiments above, obtaining access to an artery in the patient's wrist comprises obtaining access to the radial artery.

Alternatively or additionally to any of the embodiments above, advancing the deliver assist device in combination with the guide catheter over the guidewire to the desired location within the cardiac vasculature comprises advancing the deliver assist device in combination with the guide catheter over the guidewire without using an introducer sheath.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
Figure 1 is a schematic illustration of a patient;
Figure 2 is a schematic illustration of a wrist of the patient of Figure 1;
Figure 3 is a schematic illustration of a portion of the cardiac vasculature, illustrating a guidewire inserted into a cardiac artery;
Figure 4 is a schematic illustration of a portion of the cardiac vasculature, illustrating an assembly including a deliver assist device and guide catheter extended over the guidewire of Figure 3;
Figure 5 is a schematic illustration of a portion of the cardiac vasculature, illustrating a guide catheter extended over the guidewire of Figure 3 with the deliver assist device removed;
Figure 6 is a side view of a deliver assist device suitable for use in the assembly of Figure 4;
Figure 7 is a side view of a deliver assist device suitable for use in the assembly of Figure 4;
Figure 8 is a side view of the assembly of Figure 4; and
Figure 9 is a cross-sectional view of a portion of the assembly of Figure 8.

While the disclosure is amenable to various modifications and alternative forms, specifics thereof have been shown by way of example in the drawings and will be described in detail. It should be understood, however, that the intention is not to limit the invention to the particular embodiments described.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

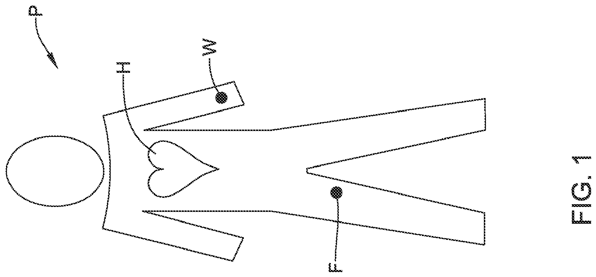

A variety of different medical interventions involve accessing the cardiac vasculature via a minimally invasive transdermal approach. Figure 1 provides a schematic illustration of a patient P having a heart H. In many cases, the heart H may be reached by entering the patient's vasculature via their femoral artery. A suitable access point for entering the femoral artery is denoted at position F. In some cases, the heart H may be reached by entering the patient's vasculature via an artery in their wrist. A suitable access point for entering an artery in the wrist is denoted at position W.

It will be appreciated that there are advantages and disadvantages to both approaches. For example, a femoral approach generally provides access to larger blood vessels, meaning that there is more room to navigate intravascular devices through the vasculature to reach the heart H. However, a femoral approach may present disadvantages with respect to controlling bleeding after the procedure has been completed because, essentially, a relatively bigger hole has been made in a relatively bigger artery.

In contrast, an approach through the wrist at position W generally has fewer potential issues for post-procedure bleeding because, essentially, a relatively smaller hole has been made in a relatively smaller artery. However, because the vasculature is generally smaller, there is less room to navigate intravascular devices through the vasculature to reach the heart H. In addition, there can be particular difficulties in navigating through the shoulder region of the patient P.

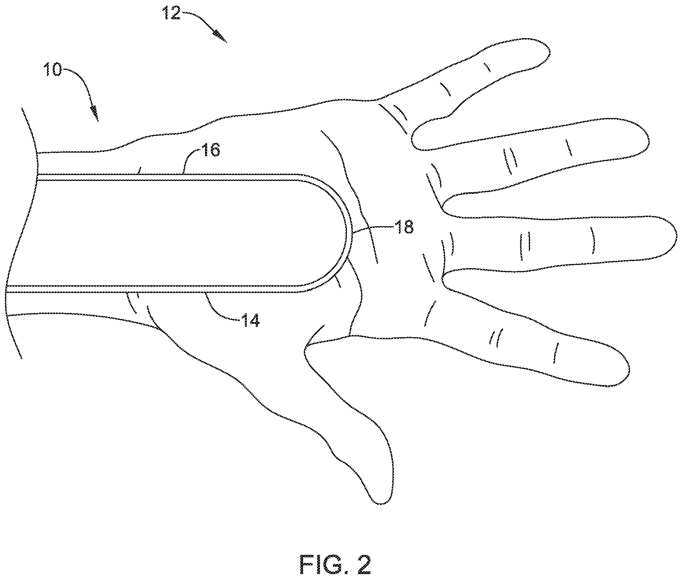

Figure 2 provides an illustration of a wrist 10 and corresponding hand 12 of the patient P. This illustration shows the inside of the wrist 10 and the palm of the hand 12. The vasculature within the wrist includes a Radial artery 14 and an Ulnar artery 16. Because the Radial artery 14 and the Ulnar artery 16 are fluidly coupled via the Superficial palmar arch 18, it will be appreciated that there is some redundancy in blood flow into the hand, which can be advantageous for several reasons. This means that blood can flow into the hand 12 even if one of the Radial artery 14 and the Ulnar artery 16 are blocked during a procedure. This also means that blood can continue to flow into the hand 12 even if one of the Radial artery 14 and the Ulnar artery 16 are damaged during a procedure.

Either the Radial artery 14 or the Ulnar artery 16 can be used. In many cases, the Radial artery 14 is used. For simplicity, use of the Radial artery 14 will be described, with the recognition that the Ulnar artery 16 may be substituted. Access to the Radial artery 14 may be gained in any of a variety of techniques, including using one or more needles of increasing diameter, introducer sheathes, and the like. In some embodiments, once access to the interior of the Radial artery 14 has been reached, additional intravascular devices such as a guidewire may be extended through the access point and into the Radial artery 14 without use of an introducer sheath. This is known as a sheathless approach.

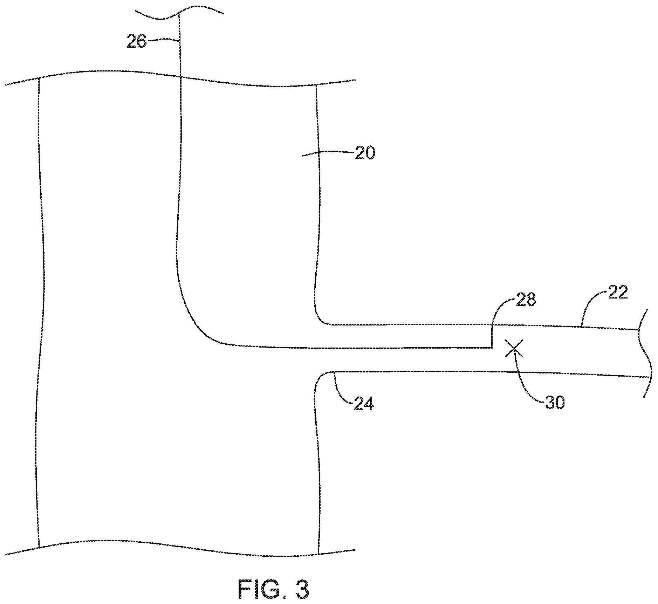

Figure 3 is a highly schematic illustration of a portion of an aorta 20 of the patient P. The illustrated portion of the aorta 20 includes a coronary artery 22 having an ostium 24. It will be appreciated that how the interior of the aorta 20 is reached may depend upon the vasculature traveled to reach the heart H, i.e., whether a femoral approach was taken or a wrist approach was taken. As illustrated, a guidewire 26 has been advanced into and through the Radial artery 14 and through the vasculature to reach the aorta 20. A distal end 28 of the guidewire 26 has been advanced into and through the ostium 24 and advanced into the coronary artery 22 to a desired location 30. While not expressly illustrated, the desired location 30 may, for example, correspond to a partial or complete vessel occlusion.

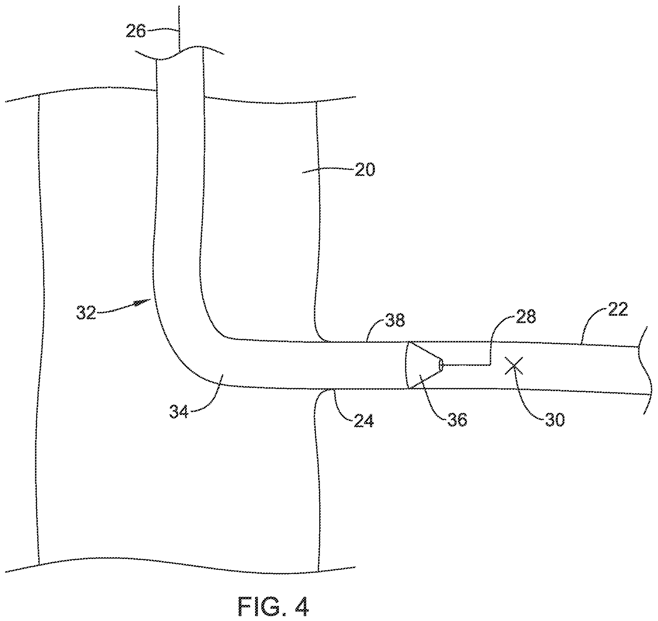

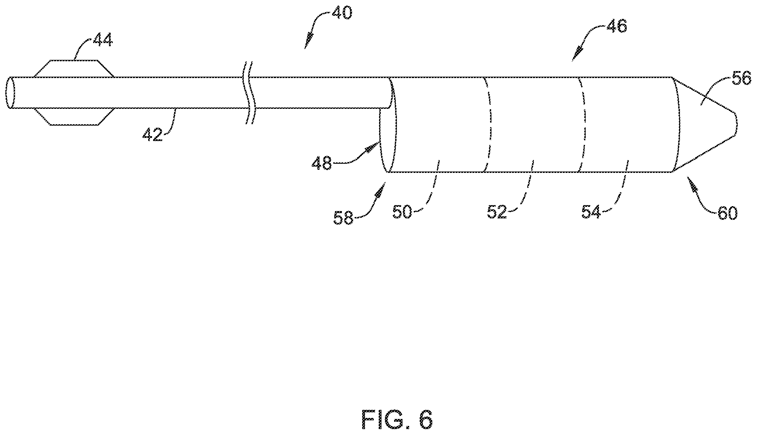

Turning to Figure 4, an assembly 32 has been advanced over the guidewire 26 to reach the desired location 30. In some embodiments, the assembly 32 includes a guide catheter 34 and a deliver assist device 36 disposed within the guide catheter 34 and extending distally from the guide catheter 34. The deliver assist device 36, which when positioned within the guide catheter 34 may be considered as increasing the stiffness and thus the pushability of the guide catheter 34, is further illustrated and described with respect to Figures 6 through 9. In some embodiments, inserting the deliver assist device 36 may alter the shape of the guide catheter 34. For example, in some cases inserting the deliver assist device 36 into the guide catheter 34 may at least partially straighten the guide catheter 34. In some cases, the deliver assist device 36 may be curved, and thus insertion of the deliver assist device 36 into the guide catheter 34 may cause the guide catheter 34 to curve.

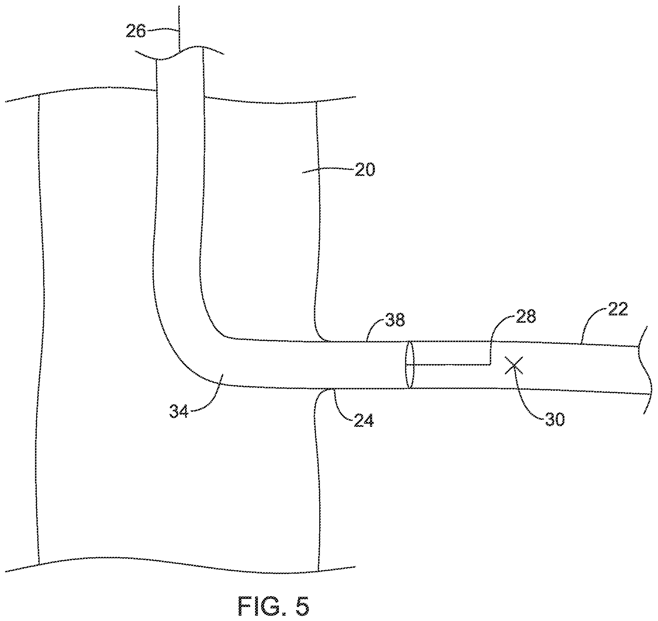

Once the assembly 32 has successfully navigated the vasculature to reach the desired location 30, the deliver assist device 36 may be removed, as illustrated in Figure 5. It can be seen that a distal end 38 of the guide catheter 34 remains positioned within the coronary artery 22 but the deliver assist device 36 has been retracted. Once the deliver assist device 36 has been removed, other therapeutic devices may be advanced through the guide catheter 34, over the guidewire 26. It should be noted that while specific reference is made herein to the guide catheter 34, in some embodiments, the guide catheter 34 may instead be a guide extension catheter. While not expressly illustrated, in some embodiments a guide extension catheter may be used in combination with the guide catheter 34 and the deliver assist device 36.

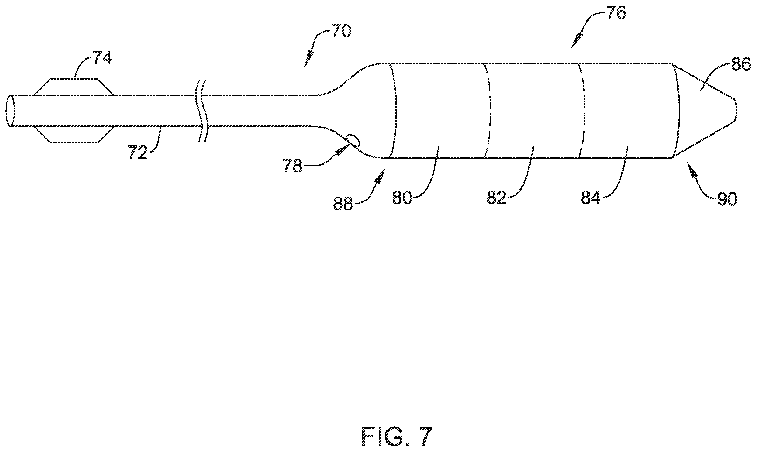

Figures 6 and 7 provide illustrations of particular deliver assist devices that could be used as part of the assembly 32. Figure 6 shows a deliver assist device 40 that includes a proximal shaft 42 extending distally from a proximal hub 44 to a flexible distal section 46. A lumen 48 can be seen as extending through the flexible distal section 46. In some embodiments, the proximal shaft 42 may be relatively stiff to improve pushability while the flexible distal section 46 is more flexible to aid in navigating turns within the vasculature. In some embodiments, the flexible distal section 46 may be considered to be a tubular structure that is secured relative to the proximal shaft 42.

In some embodiments, the flexible distal section 46 may be formed of a single material and may exhibit uniform properties such as flexibility its entire length. In some embodiments, however, the flexible distal section 46 may be formed of differing materials having differing properties such as flexibility. The flexible distal section 46 is shown schematically divided into a first portion 50, a second portion 52 and a third portion 54. A distal tip 56 may, for example, be considered as a fourth portion, or it can have properties similar to the third section 54. While three portions are illustrated, it will be appreciated that the flexible distal section 46 may be divided into any number of portions, with each portion having particular properties.

In some embodiments, there may be a distinct step-wise change in materials and/or properties between adjacent portions. In some instances, there can be material and/or property gradients between adjacent portions. In some embodiments, for example, the entire flexible distal section 46 may present a gradient in materials and properties from a proximal end 58 to a distal end 60.

The first portion 50 is formed of a polymeric material having a first durometer and the second portion 52 is formed of the same polymeric material but having a second durometer different from the first durometer. If present, the third portion 54 may be formed of the same polymeric material but have a third durometer that is different from the first durometer and the second durometer. Any suitable polymeric material having a desired durometer may be used. In an illustrative but non-limiting example, the polymeric material may be a nylon material such as a polyether block amide (PEBA), which for example is available under the trade name PEBAX®. In some embodiments, the first durometer may vary from about 20D to about 35 D, the second durometer may vary from about 35D to about 55D and the third durometer may vary from about 55D to about 80D.

Alternatively, in some embodiments, the third portion 54 may be formed of a second polymeric material different from the polymeric material of the first and second portions. A variety of different polymeric materials may be used. In some embodiments, the flexible distal section 46 may be single layer. In some embodiments, it is contemplated that the flexible distal section 46 may have a layer that extends the length of the flexible distal section 46 and a second layer formed of segments of different materials in order to provide the first, second and third portions 50, 52, 54 with the desired properties.

Figure 7 shows a deliver assist device 70 that includes a proximal shaft section 72 extending distally from a proximal hub 74 to a flexible distal section 76. In some embodiments, the flexible distal section 76 is not a separate element that is secured to the proximal shaft section 72, but rather represents a widened portion of a tubular structure forming both the proximal shaft 72 and the flexible distal section 76.

A lumen 78 can be seen as extending through the flexible distal section 76. In some embodiments, the proximal shaft 72 may be relatively stiff to improve pushability while the flexible distal section 76 is more flexible to aid in navigating turns within the vasculature. In some embodiments, the proximal shaft section 72 may, while not illustrated, include several distinct regions formed of varying materials and/or having varying properties such as flexibility.

In some embodiments, the flexible distal section 76 may be formed of a single material and may exhibit uniform properties such as flexibility its entire length. In some embodiments, however, the flexible distal section 76 may be formed of differing materials having differing properties such as flexibility. The flexible distal section 76 is shown schematically divided into a first portion 80, a second portion 82 and a third portion 84. A distal tip 86 may, for example, be considered as a fourth portion, or it can have properties similar to the third section 84. While three portion are illustrated, it will be appreciated that the flexible distal section 76 may be divided into any number of portions, with each portion having particular properties.

In some embodiments, there may be a distinct step-wise change in materials and/or properties between adjacent portions. In some instances, there can be material and/or property gradients between adjacent portions. In some embodiments, for example, the entire flexible distal section 76 may present a gradient in materials and properties from a proximal end 88 to a distal end 90.

The first portion 80 is formed of a polymeric material having a first durometer and the second portion 82 is formed of the same polymeric material but having a second durometer different from the first durometer. If present, the third portion 84 may be formed of the same polymeric material but have a third durometer that is different from the first durometer and the second durometer.

Any suitable polymeric material having a desired durometer or durometer range may be used. In an illustrative but non-limiting example, the polymeric material may be a polyether block amide (PEBA), which for example is available under the trade name PEBAX®. In some embodiments, the first durometer may vary from about 20D to about 35 D, the second durometer may vary from about 35D to about 55D and the third durometer may vary from about 55D to about 80D.

Alternatively, in some embodiments, the third portion 84 may be formed of a second polymeric material different from the polymeric material of the first and second portions. A variety of different polymeric materials may be used. In some embodiments, the flexible distal section 76 may be single layer. In some embodiments, it is contemplated that the flexible distal section 76 may have a layer that extends the length of the flexible distal section 76 and a second layer formed of segments of different materials in order to provide the first, second and third portions 80, 82, 84 with the desired properties.

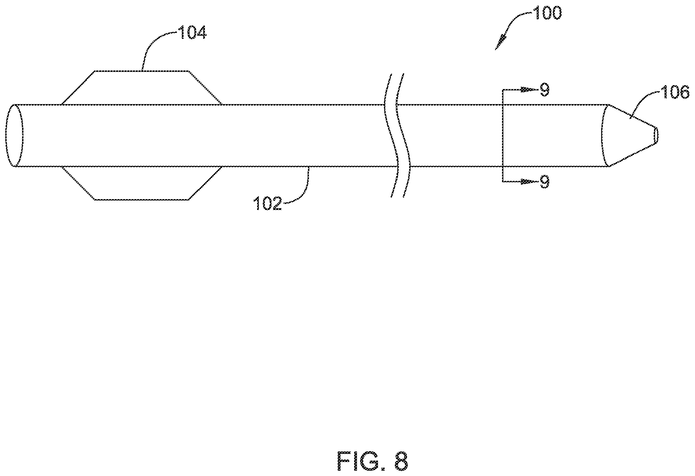

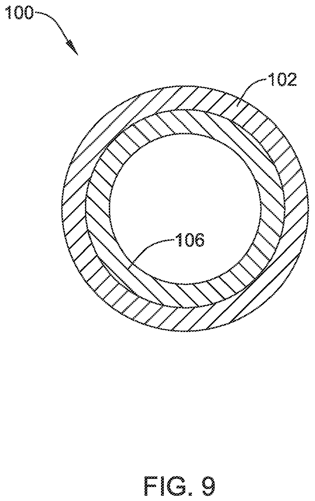

Figure 8 is a side view of an assembly 100 while Figure 9 is a cross-sectional view taken along line 9-9 of Figure 8. The assembly 100 includes a guide catheter 102 having a proximal hub 104. It will be appreciated that the guide catheter 102 may, for example, represent the guide catheter 32 shown in Figures 4 and 5. A portion of a deliver assist device 106 can be seen extending distally from the guide catheter 102. While the guide catheter 102 and the deliver assist device 106 are, with particular reference to Figure 9, each shown as being single layer, it will be appreciated that in some embodiments the guide catheter 102 and/or the deliver assist device 106 may be formed of multiple layers and a variety of different materials.

In some embodiments, some of the guidewire 26, the guide catheter 32 and 102, the deliver assist device 36, 40, 70 and 106 may include or otherwise be formed from a tubular structure that includes a plurality of slots formed within the tubular structure in order to further control flexibility properties of the aforementioned devices. Slots, if present, may be disposed at the same or a similar angle with respect to the longitudinal axis of the tubular structure bearing the slots. Slots can be disposed at an angle that is perpendicular, or substantially perpendicular, and/or can be characterized as being disposed in a plane that is normal to the longitudinal axis of a tubular structure. However, in other embodiments, slots can be disposed at an angle that is not perpendicular, and/or can be characterized as being disposed in a plane that is not normal to the longitudinal axis of a tubular structure. Additionally, a group of one or more slots may be disposed at different angles relative to another group of one or more slots. The distribution and/or configuration of slots can also include, to the extent applicable, any of those disclosed in U.S. Pat. Publication No. US 2004/01811 74.

Slots may be provided to enhance the flexibility while still allowing for suitable torque transmission characteristics. Slots may be formed such that one or more rings and/or tube segments interconnected by one or more segments and/or beams that are formed in a tubular structure, and such tube segments and beams may include portions of a tubular structure that remain after slots are formed. Such an interconnected structure may act to maintain a relatively high degree of torsional stiffness, while maintaining a desired level of lateral flexibility.

In some embodiments, some adjacent slots can be formed such that they include portions that overlap with each other. In other embodiments, some adjacent slots can be disposed such that they do not necessarily overlap with each other, but are disposed in a pattern that provides the desired degree of lateral flexibility.

Additionally, slots can be arranged along the length of, or about the circumference of, a tubular structure to achieve desired properties. For example, adjacent slots, or groups of slots, can be arranged in a symmetrical pattern, such as being disposed essentially equally on opposite sides about the circumference of a tubular structure, or can be rotated by an angle relative to each other about the axis of a tubular structure. Additionally, adjacent slots, or groups of slots, may be equally spaced along the length of a tubular structure, or can be arranged in an increasing or decreasing density pattern, or can be arranged in a non-symmetric or irregular pattern. Other characteristics, such as slot size, slot shape, and/or slot angle with respect to the longitudinal axis of a tubular structure, can also be varied.

Slots, if present, may be formed by methods such as micro-machining, saw-cutting (e.g., using a diamond grit embedded semiconductor dicing blade), electron discharge machining, grinding, milling, casting, molding, chemically etching or treating, or other known methods, and the like. Some example embodiments of appropriate micromachining methods and other cutting methods, and structures for tubular members including slots and medical devices including tubular members are disclosed in U.S. Pat. Publication Nos. 2003/0069522 and 2004/0181174-A2; and U.S. Pat. Nos. 6,766,720; and 6,5 79,246. Some example embodiments of etching processes are described in U.S. Pat. No. 5,1 06,455.

In at least some embodiments, slots may be formed in tubular member using a laser cutting process. The laser cutting process may include a suitable laser and/or laser cutting apparatus. For example, the laser cutting process may utilize a fiber laser. Utilizing processes like laser cutting may be desirable for a number of reasons. For example, laser cutting processes may allow for precise formation of slots. This may include variations in the slot width, ring width, beam height and/or width, etc. Furthermore, changes to the cutting pattern can be made without the need to replace the cutting instrument (e.g., blade).

The materials that can be used for the various components of the guidewire 26, the guide catheter 32 and 102, the deliver assist device 36, 40, 70 and 106. Some or all of these structures may include or be made from a metal, metal alloy, polymer (some examples of which are disclosed below), a metal-polymer composite, ceramics, combinations thereof, and the like, or other suitable material. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803. Other suitable materials may include ULTANIUM™ (available from Neo-Metrics) and GUM METAL™ (available from Toyota). In some other embodiments, a superelastic alloy, for example a superelastic nitinol can be used to achieve desired properties.

In at least some embodiments, portions of the aforementioned structures may also be doped with, made of, or otherwise include a radiopaque material. Radiopaque materials are understood to be materials capable of producing a relatively bright image on a fluoroscopy screen or another imaging technique during a medical procedure. This relatively bright image guides the user in determining device location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be used.

In some embodiments, a degree of Magnetic Resonance Imaging (MRI) compatibility is imparted into one or more of the guidewire 26, the guide catheter 32 and 102, the deliver assist device 36, 40, 70 and 106. For example, materials may be used that do not substantially distort the image and create substantial artifacts (e.g., gaps in the image). Certain ferromagnetic materials, for example, may not be suitable because they may create artifacts in an MRI image. Suitable materials include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

Referring now to the guidewire 26, the entire guidewire 26 may be made of the same material along its length, or in some embodiments, can include portions or sections made of different materials. In some embodiments, the material used to construct the guidewire 26 is chosen to impart varying flexibility and stiffness characteristics to different portions of the guidewire 26. For example, a proximal section and a distal section of the guidewire 26 may be formed of different materials, for example, materials having different moduli of elasticity, resulting in a difference in flexibility. In some embodiments, the material used to construct the proximal section can be relatively stiff for pushability and torqueability, and the material used to construct the distal section can be relatively flexible by comparison for better lateral trackability and steerability. For example, the proximal section can be formed of straightened 304v stainless steel wire or ribbon and the distal section can be formed of a straightened super elastic or linear elastic alloy, for example a nickel-titanium alloy wire or ribbon.

In embodiments where different portions of the guidewire 26 are made of different materials, the different portions can be connected using a suitable connecting technique and/or with a connector. For example, the different portions of the guidewire 26 can be connected using welding (including laser welding), soldering, brazing, adhesive, or the like, or combinations thereof. These techniques can be utilized regardless of whether or not a connector is utilized. The connector may include a structure generally suitable for connecting portions of a guidewire. One example of a suitable structure includes a structure such as a hypotube or a coiled wire which has an inside diameter sized appropriately to receive and connect to the ends of the proximal portion and the distal portion. Other suitable configurations and/or structures that can be utilized include those connectors described in U.S. Patent Nos. 6,918,882 and 7,071,197 and/or in U.S. Patent Pub. No. 2006-0122537.

In some embodiments, some or all of the guidewire 26, the guide catheter 32 and 102, the deliver assist device 36, 40, 70 and 106 may include or otherwise be formed of polymers. Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane (for example, Polyurethane 85A), polypropylene (PP), polyvinylchloride (PVC), polyether-ester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), poly(styrene-*b*-isobutylene-*b*-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

In some embodiments, exterior surface of the guidewire 26, the guide catheter 32 and 102, the deliver assist device 36, 40, 70 and 106 may be sandblasted, beadblasted, sodium bicarbonate-blasted, electropolished, etc. In these as well as in some other embodiments, a coating, for example a lubricious, a hydrophilic, a protective, or other type of coating may be applied. Hydrophobic coatings such as fluoropolymers provide a dry lubricity which improves guidewire handling and device exchanges. Lubricious coatings improve steerability and improve lesion crossing capability. Suitable lubricious polymers are well known in the art and may include silicone and the like, hydrophilic polymers such as high-density polyethylene (HDPE), polytetrafluoroethylene (PTFE), polyarylene oxides, polyvinylpyrrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility. Some other examples of such coatings and materials and methods used to create such coatings can be found in U.S. Patent Nos. 6,139,510 and 5,772,609.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. A deliver assist device (36) for use in combination with a guide catheter, the deliver assist device comprising:
an elongate proximal shaft (42);
a flexible distal section (46) extending distally from the elongate proximal shaft, the flexible distal section including at least a first portion (50) having a first flexibility and a second portion (52) having a second flexibility;
a lumen (48) extending through the flexible distal section, the lumen configured to accommodate a guidewire extendible therethrough; and
a tapered distal tip (56) extending distally from the flexible distal section,
wherein the first portion is formed of a polymeric material having a first durometer and the second portion is formed of the same polymeric material but having a second durometer different from the first durometer.

2. The deliver assist device of claim 1, wherein the flexible distal section further comprises a third portion (54) having a third flexibility.

3. The deliver assist device of claim 2, wherein the third portion is formed of the same polymeric material but having a third durometer different from the first durometer and the second durometer.

4. The deliver assist device of claim 2, wherein the third portion is formed of a second polymeric material different from the polymeric material of the first and second portions.

5. The deliver assist device of any of claims 1 through 4, wherein the flexible distal section is configured to provide a step-wise change in flexibility between the first portion and the second portion.

6. The deliver assist device of any of claims 1 through 5, wherein the flexible distal section comprises a tubular structure extending from the elongate proximal shaft or a widened portion of a tubular structure forming both the elongate proximal shaft and the flexible distal section.

7. The deliver assist device of any of claims 1 through 6, wherein the distal tip has a distal tip flexibility different from the first flexibility or the second flexibility.

8. The deliver assist device of claim 3, wherein the first durometer varies between about 20D to about 35 D, the second durometer varies between about 35D to about 55D, the third durometer varies from about 55D to about 80D.

9. The deliver assist device of claim 1, wherein the polymeric material is a polyether block amide (PEBA).

10. The deliver assist device of claim 1, wherein at least one of the deliver assist device and the guide catheter includes a plurality of slots.

11. An assembly comprising:
a guide catheter extending between a distal region and a proximal region;
a guide catheter lumen extending from the distal region to the proximal region; and
the deliver assist device of claim 1, wherein the device is disposable within the guide catheter lumen.

12. The assembly of claim 11, wherein the deliver assist device is configured to be disposed within the guide catheter lumen for advancement of the assembly and then be removed from the guide catheter lumen once the assembly has reached a desired location within a vasculature.

13. The assembly of any of claims 11 or 12, wherein the deliver assist device, when disposed within the guide catheter lumen for advancement of the assembly, stiffens the guide catheter.

14. The assembly of any of claims 11 through 13, wherein the deliver assist device, when disposed within the guide catheter lumen for advancement of the assembly, alters the shape of the guide catheter.

15. The assembly of claim 14, wherein insertion of the deliver assist device into the guide catheter causes the guide catheter either to straighten or to curve.

## Patentansprüche

1. Zuführhilfsvorrichtung (36) zur Verwendung in Kombination mit einem Führungskatheter,
wobei die Zuführhilfsvorrichtung aufweist:
einen langgestreckten proximalen Schaft (42);
einen flexiblen distalen Abschnitt (46), der sich distal vom langgestreckten proximalen Schaft erstreckt,
wobei der flexible distale Abschnitt mindestens einen ersten Teil (50) mit einer ersten Flexibilität und einen zweiten Teil (52) mit einer zweiten Flexibilität aufweist;
ein Lumen (48), das sich durch den flexiblen distalen Abschnitt erstreckt, wobei das Lumen zum Aufnehmen eines durch das Lumen erstreckbaren Führungsdrahts ausgebildet ist; und
eine konische distale Spitze (56), die sich distal vom flexiblen distalen Abschnitt aus erstreckt,
wobei der erste Teil aus einem Polymermaterial mit einem ersten Härtegrad gebildet ist und der zweite Abschnitt aus dem gleichen Polymermaterial gebildet ist, jedoch einen vom ersten Härtegrad verschiedenen zweiten Härtegrad aufweist.

2. Zuführhilfsvorrichtung nach Anspruch 1, wobei der flexible distale Abschnitt ferner einen dritten Teil (54) mit einer dritten Flexibilität aufweist.

3. Zuführhilfsvorrichtung nach Anspruch 2, wobei der dritte Teil aus dem gleichen Polymermaterial gebildet ist, jedoch einen dritten Härtegrad aufweist, der vom ersten Härtegrad und vom zweiten Härtegrad verschieden ist.

4. Zuführhilfsvorrichtung nach Anspruch 2, wobei der dritte Teil aus einem zweiten Polymermaterial gebildet ist, das von dem Polymermaterial des ersten und zweiten Teils verschieden ist.

5. Zuführhilfsvorrichtung nach einem der Ansprüche 1 bis 4, wobei der flexible distale Abschnitt zum Vorsehen einer schrittweisen Veränderung der Flexibilität zwischen dem ersten Teil und dem zweiten Teil ausgebildet ist.

6. Zuführhilfsvorrichtung nach einem der Ansprüche 1 bis 5, wobei der flexible distale Abschnitt eine Rohrstruktur, die sich vom langgestreckten proximalen Schaft erstreckt, oder einen aufgeweiteten Teil einer Rohrstruktur, die sowohl den langgestreckten proximalen Schaft als auch den flexiblen distalen Abschnitt bildet, aufweist.

7. Zuführhilfsvorrichtung nach einem der Ansprüche 1 bis 6, wobei die distale Spitze eine von der ersten Flexibilität oder der zweiten Flexibilität verschiedene Distalspitzenflexibilität aufweist.

8. Zuführhilfsvorrichtung nach Anspruch 3, wobei der erste Härtegrad von etwa 20D bis etwa 35D, der zweite Härtegrad von etwa 35D bis etwa 55D, der dritte Härtegrad von etwa 55D bis etwa 80D variiert.

9. Zuführhilfsvorrichtung nach Anspruch 1, wobei das Polymermaterial ein Polyether-Blockamid (PEBA) ist.

10. Zuführhilfsvorrichtung nach Anspruch 1, wobei die Zuführhilfsvorrichtung und/oder der Führungskatheter mehrere Schlitze aufweist.

11. Baugruppe, aufweisend:
einen Führungskatheter, der sich zwischen einem distalen Bereich und einem proximalen Bereich erstreckt;
ein Führungskatheterlumen, das sich von dem distalen Bereich zum proximalen Bereich erstreckt; und
die Zuführhilfsvorrichtung nach Anspruch 1, wobei die Vorrichtung im Führungskatheterlumen anordenbar ist.

12. Baugruppe nach Anspruch 11, wobei die Zuführhilfsvorrichtung dazu ausgebildet ist, zum Vorschieben der Baugruppe im Führungskatheterlumen angeordnet zu sein und dann aus dem Führungskatheterlumen entfernt zu werden, wenn die Baugruppe einen gewünschten Ort in einem Gefäßsystem erreicht hat.

13. Baugruppe nach Anspruch 11 oder 12, wobei die Zuführhilfsvorrichtung den Führungskatheter versteift, wenn sie zum Vorschieben der Baugruppe im Führungskatheterlumen angeordnet ist.

14. Baugruppe nach einem der Ansprüche 11 bis 13, wobei die Zuführhilfsvorrichtung die Form des Führungskatheters verändert, wenn sie zum Vorschieben der Baugruppe im Führungskatheterlumen angeordnet ist.

15. Baugruppe nach Anspruch 14, wobei das Einführen der Zuführhilfsvorrichtung in den Führungskatheter den Führungskatheter dazu veranlasst, sich entweder geradezubiegen oder sich zu krümmen.

## Revendications

1. Dispositif d'assistance de pose (36) destiné à être utilisé en combinaison avec un cathéter de guidage, le dispositif d'assistance de pose comprenant :
une tige proximale allongée (42) ;
une section distale flexible (46) s'étendant de manière distale à partir de la tige proximale allongée,
la section distale flexible comprenant au moins une première partie (50) ayant une première flexibilité et une deuxième partie (52) ayant une deuxième flexibilité ;
une lumière (48) s'étendant à travers la section distale flexible, la lumière étant configurée pour loger un fil-guide extensible à travers cette dernière ; et
une pointe distale progressivement rétrécie (56) s'étendant de manière distale à partir de la section distale flexible,
dans lequel la première partie est formée avec un matériau polymère ayant un premier duromètre et la deuxième partie est formée avec le même matériau polymère mais ayant un deuxième duromètre différent du premier duromètre.

2. Dispositif d'assistance de pose selon la revendication 1, dans lequel la section distale flexible comprend en outre une troisième partie (54) ayant une troisième flexibilité.

3. Dispositif d'assistance de pose selon la revendication 2, dans lequel la troisième partie est formée avec le même matériau polymère mais ayant un troisième duromètre différent du premier duromètre et du deuxième duromètre.

4. Dispositif d'assistance de pose selon la revendication 2, dans lequel la troisième partie est formée avec un second matériau polymère différent du matériau polymère des première et deuxième parties.

5. Dispositif d'assistance de pose selon l'une quelconque des revendications 1 à 4, dans lequel la section distale flexible est configurée pour fournir un changement par palier du point de vue de la flexibilité entre la première partie et la deuxième partie.

6. Dispositif d'assistance de pose selon l'une quelconque des revendications 1 à 5, dans lequel la section distale flexible comprend une structure tubulaire s'étendant à partir de la tige proximale allongée ou une partie élargie d'une structure tubulaire formant à la fois la tige proximale allongée et la section distale flexible.

7. Dispositif d'assistance de pose selon l'une quelconque des revendications 1 à 6, dans lequel la pointe distale a une flexibilité de pointe distale différente de la première flexibilité ou de la deuxième flexibilité.

8. Dispositif d'assistance de pose selon la revendication 3, dans lequel le premier duromètre varie entre environ 20D et environ 35D, le deuxième duromètre varie entre environ 35D et environ 55D, le troisième duromètre varie d'environ 55D et environ 80D.

9. Dispositif d'assistance de pose selon la revendication 1, dans lequel le matériau polymère est un polyéther bloc amide (PEBA).

10. Dispositif d'assistance de pose selon la revendication 1, dans lequel au moins l'un parmi le dispositif d'assistance de pose et le cathéter de guidage comprend une pluralité de fentes.

11. Ensemble comprenant :
un cathéter de guidage s'étendant entre une région distale et une région proximale ;
une lumière de cathéter de guidage s'étendant à partir de la région distale jusqu'à la région proximale ; et
le dispositif d'assistance de pose selon la revendication 1, dans lequel le dispositif peut être disposé dans la lumière du cathéter de guidage.

12. Ensemble selon la revendication 11, dans lequel le dispositif d'assistance de pose est configuré pour être disposé dans la lumière du cathéter de guidage pour faire avancer l'ensemble et être ensuite retiré de la lumière du cathéter de guidage une fois que l'ensemble a atteint un emplacement souhaité dans un système vasculaire.

13. Ensemble selon l'une quelconque des revendications 11 ou 12, dans lequel le dispositif d'assistance de pose, lorsqu'il est disposé dans la lumière du cathéter de guidage pour l'avancement de l'ensemble, renforce le cathéter de guidage.

14. Ensemble selon l'une quelconque des revendications 11 à 13, dans lequel le dispositif d'assistance de pose, lorsqu'il est disposé dans la lumière du cathéter de guidage pour l'avancement de l'ensemble, modifie la forme du cathéter de guidage.

15. Ensemble selon la revendication 14, dans lequel l'insertion du dispositif d'assistance de pose dans le cathéter de guidage amène le cathéter de guidage à se redresser ou à s'incurver.
